(19) **Europäisches Patentamt / European Patent Office / Office européen des brevets**

(11) **EP 2 988 131 A1**

(12) **EUROPEAN PATENT APPLICATION**
published in accordance with Art. 153(4) EPC

(43) Date of publication:
**24.02.2016 Bulletin 2016/08**

(21) Application number: **14785227.1**

(22) Date of filing: **18.04.2014**

(51) Int Cl.:
*G01N 33/574* (2006.01)    *C12Q 1/68* (2006.01)
*G01N 33/53* (2006.01)

(86) International application number:
**PCT/KR2014/003384**

(87) International publication number:
**WO 2014/171778 (23.10.2014 Gazette 2014/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.04.2013 KR 20130043160**

(71) Applicant: **Gencurix Inc.**
**Seoul 152-733 (KR)**

(72) Inventors:
• **SHIN, Young Kee**
**Seoul 135-968 (KR)**

• **KIM, Young-Deug**
**Incheon 402-715 (KR)**
• **OH, Eu Sel**
**Seoul 137-830 (KR)**
• **CHOI, Jun Young**
**Gwangmyeong-si**
**Gyeonggi-do 423-762 (KR)**
• **CHO, Sang Rea**
**Seoul 110-033 (KR)**

(74) Representative: **Stolmár & Partner**
**Patentanwälte PartG mbB**
**Blumenstraße 17**
**80331 München (DE)**

(54) **GENETIC MARKER FOR EARLY BREAST CANCER PROGNOSIS PREDICTION AND DIAGNOSIS, AND USE THEREOF**

(57) The present invention relates to a gene for predicting or diagnosing the prognosis of early-stage breast cancer and to a use thereof, and more specifically relates to a genetic marker for predicting or diagnosing the prognosis of breast cancer, including TRBC1 (T cell receptor beta constant 1), BTN3A2 (butyrophilin, subfamily 3, member A2) or HLA-DPA1 (major histocompatibility complex, class II, DP alpha 1) for providing information necessary for predicting or diagnosing the prognosis of a breast cancer patient. The genetic marker of the present invention allows the prediction or diagnosis of the prognosis of a breast cancer patient, and can therefore advantageously be used for the purpose of providing a directon as to the future course of breast cancer treatment, including a decision on whether anticancer therapy is necessary.

【FIG. 7a】

**Description**

[Technical Field]

The present application claims priority from Korean Patent Application No. 10-2013-0043160 filed on April 18 2013, the disclosure of which is incorporated herein by reference in its entirety.
The present invention relates to a gene for early-stage breast cancer prognosis prediction and diagnosis and a use thereof, and more specifically, to a genetic marker for early-stage breast cancer prognosis prediction and diagnosis, of TRBC1 (T cell receptor beta constant 1), BTN3A2 (butyrophilin, subfamily 3, member A2), or HLA-DPA1 (major histocompatibility complex, class II, DP alpha 1), for providing information necessary for the prognosis prediction and diagnosis of a breast cancer patient, and to a use thereof.

[Background Art]

As human genome information has been actively utilized, cancer research has focused on the establishment of mechanisms at the genome level. Particularly, cancer cell characteristics can be identified in a macroscopic view, based on information about expression patterns of tens of thousands of genes or on the increase or decrease in the number of genes using microarrays. This analysis of the genome-level information is very innovative in understanding organic and complicated life phenomena, and will be more commonly used. Specifically, in cases of complex diseases such as cancer, the analysis of a small number of particular genes is likely to obtain narrow results, and it is important to capture large behavior patterns with respect to the occurrence and development of cancer, and thus genome information analysis is absolutely necessary. As described above, most of the genome information that is a basis for cancer research is created using genome chips such as a microarray, and technologies that can obtain a lot of information at once are evolving day by day. In spite of the disadvantages of high costs, research using microarrays is being actively developed, so the amount of related information is explosively increasing. Since the mid-2000s, such genome information has started to be collected and made into a database, and secondary and tertiary analysis using the information thus obtained is becoming a focal point for the research of life phenomena.
Tens of thousands of probes indicating approximately 20,000 to 30,000 genes are embedded in general expression gene chips, and more than one million probes are often embedded in microarrays that measure precise information, such as SNP. Methods using these microarrays are very efficient since they are relatively simple and standardized and a large amount of information can be obtained at once in a short time, but analyzing the obtained results is a key point as well as being a difficult bottleneck. Comprehensive analysis for tens of thousands of genes incomparable to existing analysis for a small number of genes must be supported by a broad knowledge of the genome as well as statistical analysis techniques, so useful information can be eventually obtained. Besides, high-performance computing equipment capable of storing and analyzing large amounts of information are needed, and the related computational techniques are also needed. Meanwhile, it is difficult to perform for the researchers who are familiar with conventional biological research ranges and experimental methods, and thus, the methods cannot be favorably utilized even though genome information increases at an extraordinary rate in Korea. Considering the domestic situation with respect to capital and technology research that are insufficient compared with North America and Europe, actively utilizing known genome information should be at the head of bioinformatics. Genome analysis has been actively introduced in the research of, particularly, cancers, and a considerable amount of related-information has been accumulated.
Breast cancer is frequently detected in the early stage since self-diagnosis is possible and the importance of self-diagnosis is highly publicized. It was difficult to determine whether early-stage breast cancer patients should be allowed to receive anticancer treatments after surgery. It is possible to roughly predict a prognosis through pathological observation, but the observation result is difficult to normalize and quantify, and the reliability on prognosis prediction is low, and thus, most of early-stage breast cancer patients are recommended to receive anticancer treatments in actual clinical practice. Due to the nature of anticancer treatments, they are very expensive, while the patients suffer from very serious pains. It is estimated that more than half of early-stage breast cancer patients do not need to receive anticancer treatments. Therefore, if unnecessary anticancer treatments are reduced by analyzing the characteristics of the early-stage breast cancer to predict prognosis of patient, it may be a great help to increase the quality of life of the patients. As the information about tens of thousands of breast cancer gene expression patterns is obtained at once using microarrays, research for classifying breast cancer types at the molecular level and establishing mechanisms of cancer occurrence and development are being actively conducted. It is important to predict the prognosis of the early-stage breast cancer patients in clinical practice. The work of identifying genes for prognosis prediction using microarrays has already started in the early 2000s. Although research that uses microarrays is expensive, a significant number of breast cancer tissue expression profiles have been produced and available to researchers. Starting from the identifying of 70 prognosis prediction genes by analyzing the early-stage breast cancer tissues and survival data of 78 patients followed up for 10 years in 2002, a dozen genes for prognosis prediction genes were then published, and among these genes, several genes have already been commercialized and used in clinical practice (Chang, H.Y., et al., Gene expression signature of fibroblast serum response predicts human cancer progression: similarities between tumors and wounds. PLoSBiol 2(2): p. E7(2004); van de Vijver, M.J., et al., A gene-expression signature as a predictor of survival in breast cancer. N Engl J Med 347(25):1999-2009(2002); van 't Veer, L.J., et al., Gene expression profiling predicts clinical outcome of breast cancer. Nature 415(6871): 530-536(2002); Wang, Y., et al., Gene-expression profiles to predict distant metastasis of lymph-node-negative primary breast cancer. Lancet 365(9460): 671-679(2005); Buyse, M., et al., Validation and clinical utility of a 70-gene prognostic signature for women with node-negative breast cancer. J Natl Cancer Inst, 98(17):1183-92(2006); Paik, S., Development and clinical utility of a 21-gene recurrence score prognostic assay in patients with early-stage breast cancer treated with tamoxifen. Oncologist 12 (6) :631-635(2007); Paik, S. , et al. , A multigene assay to predict recurrence of tamoxifen-treated, node-negative breast cancer. N Engl J Med 351(27) :2817-2826(2004); Sotiriou, C., et al., Gene expression profiling in breast cancer: understanding the molecular basis of histologic grade to improve prognosis. J Natl Cancer Inst 98(4):262-72(2006); Pawitan, Y., et al., Gene expression profiling spares early-stage breast cancer patients from adjuvant therapy: derived and validated in two population-based cohorts. Breast Cancer Res 7(6):R953-964(2005); Miller, L.D., et al., An expression signature for p53 status in human breast cancer predicts mutation status, transcriptional effects, and patient survival. Proc Natl AcadSci USA, 102 (38) :13550-13555(2005); Bild, A.H., et al., Oncogenic pathway signatures in human cancers as a guide to targeted therapies. Nature 439(7074):353-357(2006); Teschendorff, A.E., et al., A consensus prognostic gene expression classifier for ER positive breast cancer. Genome Biol 7(10):R101(2006); Desmedt, C., et al., Strong time dependence of the 76-gene prognostic signature for node-negative breast cancer patients in the TRANSBIG multicenter independent validation series. Clin Cancer Res 13(11): 3207-3214(2007)). Representative examples thereof are MammaPrint (Agendia) and Oncotype DX (Genomic Health), which are being currently used in clinical practice. However, they have been used as one of the references for prognosis (van de Vijver, M.J., et al., A gene-expression signature as a predictor of survival in breast cancer. N Engl J Med 347(25):1999-2009(2002); Paik, S., et al., A multigene assay to predict recurrence of tamoxifen-treated, node-negative breast cancer. N Engl J Med 351(27) :2817-2826(2004)).

Throughout the entire specification, many research papers and patent documents are referenced and their citations are disclosed. The disclosure of cited papers and patent documents are entirely incorporated by reference into the present specification, and the level of the technical field within which the present invention falls, and details of the present invention are explained more clearly.

[Detailed Description of the Invention]

[Technical Problem]

The present inventors have endeavored to develop a gene diagnosis system capable of predicting the prognosis of the early-stage breast cancer patient and determining whether anticancer treatment is performed on the early-stage breast cancer patient by using an FFPE sample of the tissue containing cancer cells of the patient. As a result, the present inventors have identified genes associated with prognosis prediction by collecting and analyzing microarray data and clinical information, which are obtained from the early-stage breast cancer tissue; selected genes and sets thereof, which are suitable for the application to the FFPE sample, among the identified genes; and validated utility of the selected genes and gene sets, thereby completing the present invention.

Therefore, an aspect of the present invention is to provide a genetic marker for predicting or diagnosing the prognosis of a breast cancer patient, and a use thereof.

Another aspect of the present invention is to provide a novel method for predicting or diagnosing the prognosis of a breast cancer patient.

Still another aspect of the present invention is to provide a kit for predicting or diagnosing the prognosis of a breast cancer patient.

Still another aspect of the present invention is to provide a method for calculating a breast cancer prognosis predictive value in order to provide information necessary for predicting or diagnosing the prognosis of a breast cancer patient, the method comprising isolating mRNA from a patient sample, measuring the gene expression level, normalizing the gene expression level, and calculating a predictive value.

[Technical Solution]

In accordance with an aspect of the present invention, there is provided a genetic marker for predicting or diagnosing the prognosis of a breast cancer patient and a use thereof.

In accordance with another aspect of the present invention, there is provided a novel method for predicting or diagnosing the prognosis of a breast cancer patient.

In accordance with still another aspect of the present invention, there is provided a kit for predicting or diagnosing the prognosis of a breast cancer patient.

In accordance with still another aspect of the present invention, there is provided a method for calculating a predictive value of the prognosis of breast cancer to provide information necessary for predicting or diagnosing the prognosis of a breast cancer patient, the method comprising isolating mRNA from a patient sample, measuring a gene expression level, normalizing the gene expression level, and calculating a predictive value.

In accordance with still another aspect of the present invention, there is provided a primer pair for at least one gene selected from the group consisting of T cell receptor beta constant 1 (TRBC1), butyrophilin, subfamily 3, member A2 (BTN3A2), and major histocompatibility complex, class II, DP alpha 1 (HLA-DPA1), wherein the primer pair is capable of amplifying a target gene through PCR amplification.

In accordance with still another aspect of the present invention, there is provided a use of a primer pair for preparing an agent for predicting the prognosis of breast cancer, wherein the primer pair is for at least one gene selected from the group consisting of TRBC1, BTN3A2, and HLA-DPA1, and wherein the primer pair is capable of amplifying a target gene through PCR amplification.

Unless defined otherwise, all technical and scientific terms used herein have the same meanings as are commonly understood by a person skilled in the art. The following reference documents provide one of skills that have general definitions of many terms used herein: Singleton et al., DICTIONARY OF MICROBIOLOGY AND MOLECULAR BIOLOTY (2ded.1994); THE CAMBRIDGE DICTIONARY OF SCIENCE AND TECHNOLOGY (Walkered.,1988); and Hale &Marham, THE HARPER COLLINS DICTIONARY OF BIOLOGY.

Hereinafter, the present invention will be described in more detail.

The present invention provides a genetic marker for predicting or diagnosing the prognosis of a breast cancer patient and a use thereof. More specifically, the present invention provides a genetic marker of T cell receptor beta constant 1 (TRBC1), butyrophilin, subfamily 3, member A2 (BTN3A2), or major histocompatibility complex, class II, DP alpha 1 (HLA-DPA1), for predicting or diagnosing the prognosis of breast cancer, especially, early-stage breast cancer. In addition, the present invention provides a method for predicting the prognosis of breast cancer, the method comprising: (a) isolating mRNA from a sample; (b) measuring the mRNA expression level of at least one gene selected from the group consisting of T cell receptor beta constant 1 (TRBC1), butyrophilin, subfamily 3, member A2 (BTN3A2), and major histocompatibility complex, class II, DP alpha 1 (HLA-DPA1); and (c) normalizing the mRNA expression level of the gene, wherein the overexpression of the gene is determined as indicating good prognosis.

Herein, T cell receptor beta constant 1 (TRBC1), butyrophilin, subfamily 3, member A2 (BTN3A2), or major histocompatibility complex, class II, DP alpha 1 (HLA-DPA1) may serve as a genetic marker in the present invention. They may be used for predicting or diagnosing the prognosis of early-stage breast cancer by being independently selected, or by a combination of two or three genes. Each gene may be a sequence thereof known in the art or a sequence of the synonym thereof, and preferably a sequence thereof derived from a human being. More preferably, TRBC1 may be Genbank Accession No. BC030533.1; BTN3A2 may be Genbank Accession No. NM_007047.3; and HLA-DPA1 may be Genbank Accession No. N_001242524.1, NM_001242525.1, or NM_033554.3. The synonym and the sequence of each gene may be searched in Genbank or Swissprot.

Herein, the breast cancer may be an invasive breast cancer, or breast cancer stage I, II, or III. Herein, the breast cancer may be estrogen receptor positive (ER+).

As used herein, the term "prognosis" refers to symptoms in the future or prospects of progress determined by disease diagnose. The prognosis in cancer patients generally means whether or not the cancer is metastatic within a certain period or the survival period after the occurrence of cancer or the surgical procedure. The prediction of prognosis (or the diagnosis of prognosis) presents clues for future direction of breast cancer treatment, especially, including whether chemotherapy treatment is performed on early-stage breast cancer patients, and thus is a very important clinical challenge. The prediction of prognosis includes predictions of the response of patients to disease therapeutic agents and therapeutic progress.

Herein, the sample may be a breast cancer tissue of a breast cancer patient. The breast cancer tissue may contain some normal cells, and may preferably be a formalin-fixed paraffin-embedded (FFPE) sample of the breast cancer tissue including cancer cells of the patient.

The marker for predicting or diagnosing the prognosis of breast cancer according to the present invention may be detected through polymerase chain reaction (PCR) amplification of a target gene. The detection of the target gene according to the present invention is preferably a detection of the expression level of the target gene, and more preferably a quantitative detection of the expression level of the target gene. For the detection of the expression level, the isolation of mRNA in the sample tissue and the synthesis of cDNA from the mRNA may be needed. For the isolation of mRNA, the isolation methods of mRNA from a sample, which are known in the art, may be employed, and since the sample is preferably an FFPE sample, the isolation methods of mRNA, which are appropriate for the FFPE sample, may be employed. For the synthesis of cDNA, the cDNA synthesis methods using mRNA as a template, which are known in the art, may be employed. Preferably, the detection of the marker for predicting or diagnosing the prognosis of breast cancer according to the present invention is a quantitative detection of the mRNA expression in the FFPE sample, and thus may be the detection by the isolation method of mRNA and the reverse transcription quantitative polymerase chain reaction (RT-qPCR) method with respect to the FFPE sample.

Herein, the detection may be a measurement of the mRNA expression level. The measurement of the expression level may be conducted using the methods known in the art, but may be conducted by an optical quantitative analysis system using a probe labeled with a reporter fluorescent dye and/or a quencher fluorescent dye. The measurement may be conducted using a commercially available system, for example, ABI PRISM 7700™ Sequence Detection System™, Roche Molecular BiochemicalsLightcycler, and an operating system affiliated therewith, such as software. The measurement data may be expressed as a measurement value or a threshold cycle (Ct or Cp). The point in which the measured fluorescent value is first recorded as being statistically significant is defined as the threshold cycle. The threshold cycle is inversely proportional to the value at the beginning in which targets of detection are present as a template of PCR, and thus the lower threshold cycle value indicates the presence of the quantitatively increased targets of detection.

Meanwhile, the present invention provides a composition for predicting or diagnosing the prognosis of breast cancer, the composition comprising a primer pair as an active ingredient, wherein the primer pair is for at least one gene selected from the group consisting of TRBC1, BTN3A2, and HLA-DPA1, and wherein the primer pair is capable of amplifying a target gene through PCR amplification.

As used herein, the term "primer" refers to an oligonucleotide, and the primer may act as an initial point of synthesis in the condition where the synthesis of the primer extension products that are complementary to a nucleic acid chain (template) is induced, that is, the presence of nucleotides and polymerases such as DNA polymerases, and appropriate temperature and pH. Preferably, the primer is deoxyribonucleotide, and has a single chain. The primer used herein may include naturally occurring dNMPs (that is, dAMP, dGMP, dCMP, and dTMP), modified nucleotides, or non-naturally occurring nucleotides. Also, the primer may include ribonucleotides.

The primer of the present invention may be an extension primer that is annealed to a target nucleic acid to form a sequence complementary to the target nucleic acid by template-dependent nucleic acid polymerase. The extension primer is extended to a site at which an immobilized probe is annealed, and thus occupies the site at which the probe is annealed.

The extension primer used herein includes a hybrid nucleotide sequence complementary to the first site of the target nucleic acid. The term "complementary" refers to being sufficiently complementary such that primers or probes are selectively hybridized with the target nucleic acid sequence under predetermined annealing or hybridizing conditions, encompassing the terms "substantially complementary" and "perfectly complementary", and means preferably "perfectly complementary". As used herein, the term "substantially complementary sequence" in conjunction with the primer sequence, means including the sequence that is partially un-identical to the sequence of the comparative target within the range in which the sequence is annealed to a particular sequence to serve as a primer, as well as the perfectly identical sequence.

The primer needs to be long enough to prime the synthesis of extension products in the presence of polymerases. The appropriate length of the primer varies depending on several factors, such as temperature, field of application, and primer source, but the primer generally has 15-30 nucleotides. Short primer molecules generally require lower temperatures in order to form sufficiently stable hybrid complexes together with templates. The term "annealing" or "priming" refers to the apposition of oligodeoxynucleotide or nucleic acid to the template nucleic acid, and the apposition enables the polymerase to polymerize nucleotides to form a nucleic acid molecule, which is complementary to the template nucleic acid or a portion thereof.

The sequences of primers do not need to have a perfectly complementary sequence to some sequences of templates. The primers are good enough so long as they have sufficient complementarity within the scope in which they can perform their inherent actions through hybridization with the template. Therefore, the primer of the present invention does not need to have a perfectly complementary sequence to the foregoing nucleotide sequence as a template. The primers are good enough so long as they have sufficient complementarity within the scope in which they can perform their actions through hybridization with the gene sequence. This design of the primers may be easily carried out by a person skilled in the art with reference to the foregoing nucleotide sequences. For instance, the design of the primers may be carried out using computer programs for primer design (e.g., PRIMER 3 program).

The present invention provides a kit for predicting or diagnosing the prognosis of breast cancer, the kit comprising the primer pair. The kit of the present invention may further comprise tools and/or reagents known in the art which are used for PCR, RNA separation in samples, and cDNA synthesis, in addition to primer pairs allowing PCR amplification of TRBC1, BTN3A2, and/or HLA-DPA1. The kit of the present invention may further comprise, if necessary, tubes which are to be used to mix respective components, well plates, instruction manuals describing how to use, or the like.

The present invention provides a method for calculating a predictive value of the prognosis of breast cancer to provide information necessary for predicting or diagnosing the prognosis of a breast cancer patient, the method comprising:

(a) isolating mRNA from a sample;

(b) measuring the mRNA expression level of at least one gene selected from the group consisting of T cell receptor beta constant 1 (TRBC1), butyrophilin, subfamily 3, member A2 (BTN3A2), and major histocompatibility complex, class II, DP alpha 1 (HLA-DPA1);

(c) normalizing the mRNA expression level of the gene;

(d) inserting the normalized value into a predetermined calculation formula to calculate a numerical value; and

(e) determining the breast cancer prognosis as being good(favorable) or poor(unfavorable) depending on the numerical value.

Herein, the expression level of the target gene for detection needs to be normalized since the overall gene expression level may vary depending on the target patient or sample. The normalization is made through the difference in the expression of the gene, which can indicate the difference from the basic expression level, and preferably, the normalization may be carried out by measuring the expression levels of one to five genes selected from C-terminal-binding protein 1 (CTBP1), TATA-binding protein (TBP), hydroxymethylbilane synthase (HMBS), cullin 1 (CUL1), and ubiquilin-1 (UBQLN1) (or the mean of expression levels of selected multiple genes), and calculating the ratio thereof.

Meanwhile, the present invention provides a method for breast cancer prognosis prediction and diagnosis, the method comprising a step of using a primer pair to measure the mRNA expression level of a selected gene from a sample of a breast cancer patient,

wherein the primer pair is for at least one gene selected from the group consisting of TRBC1, BTN3A2, and HLA-DPA1 and wherein the primer pair is capable of amplifying a target gene through PCR amplification.

Furthermore, the present invention provides a primer pair for at least one gene selected from the group consisting of TRBC1, BTN3A2, and HLA-DPA1, wherein the primer pair is capable of amplifying a target gene through PCR amplification.

Still furthermore, the present invention provides a use of a primer pair for preparing a preparation for predicting the prognosis of breast cancer, wherein the primer pair is for at least one gene selected from the group consisting of TRBC1, BTN3A2, and HLA-DPA1, and wherein the primer pair is capable of amplifying a target gene through PCR amplification.

The following references are made in the above-mentioned nucleotide and protein works (Maniatis et al., Molecular Cloning: A Laboratory Manual, Cold Spring Harbor Laboratory, Cold Spring Harbor, N.Y.(1982); Sambrook et al., Molecular Cloning: A Laboratory Manual, 2d Ed., Cold Spring Harbor Laboratory Press(1989); Deutscher, M., Guide to Protein Purification Methods Enzymology, vol. 182. Academic Press. Inc., San Diego, CA(1990); Ausubel et al., Current Protocols of Molecular Biology, John Wiley and Sons (1997); Rupp and Locker, Lab Invest. 56: A67 (1987); De Andres et al., BioTechniques 18: 42044 (1995); Held et al., Genome Research 6:986-994 (1996); T.E. Godfrey et al. J. Molec. Diagnostics 2: 84-91 (2000); K. Specht et al., Am. J. Pathol. 158: 419-29 (2001)).

[Advantageous Effects]

Accordingly, the present invention provides a genetic marker for predicting or diagnosing the prognosis of early-stage breast cancer. The genetic marker of the present invention enables the prediction or diagnosis of the prognosis of a breast cancer patient, and thus can be favorably used to present clues for the future direction of breast cancer treatment, including the determination on whether anticancer treatment is needed.

[Brief Description of the Drawings]

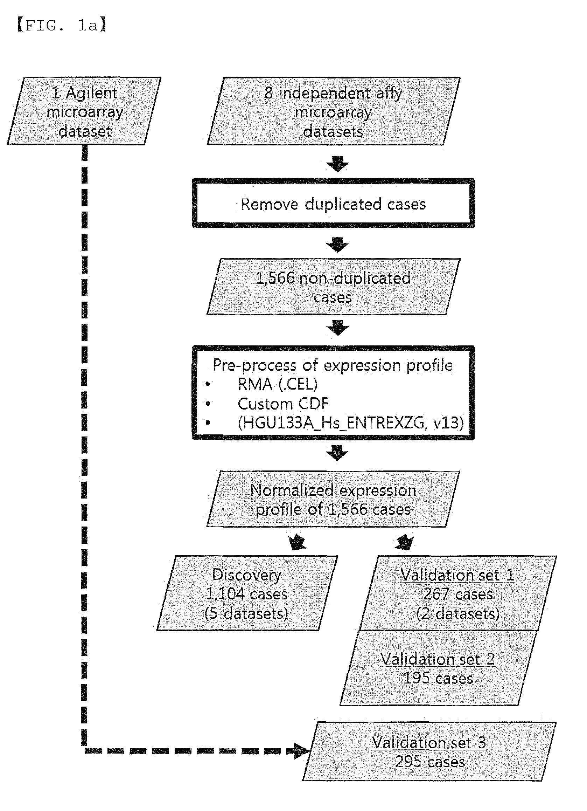

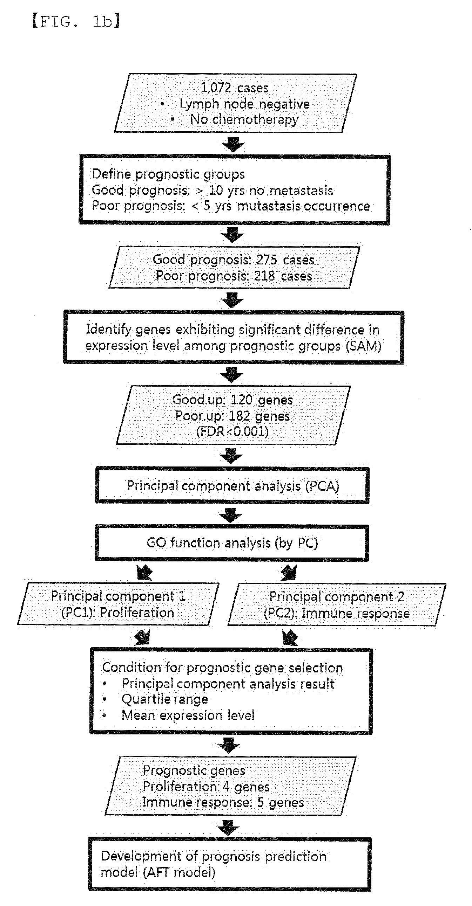

FIG. 1a schematically shows a normalization procedure by curation and pre-processing of microarray data of breast cancer tissue. FIG. 1b schematically shows an identification procedure of prognostic genes in the discovery dataset.

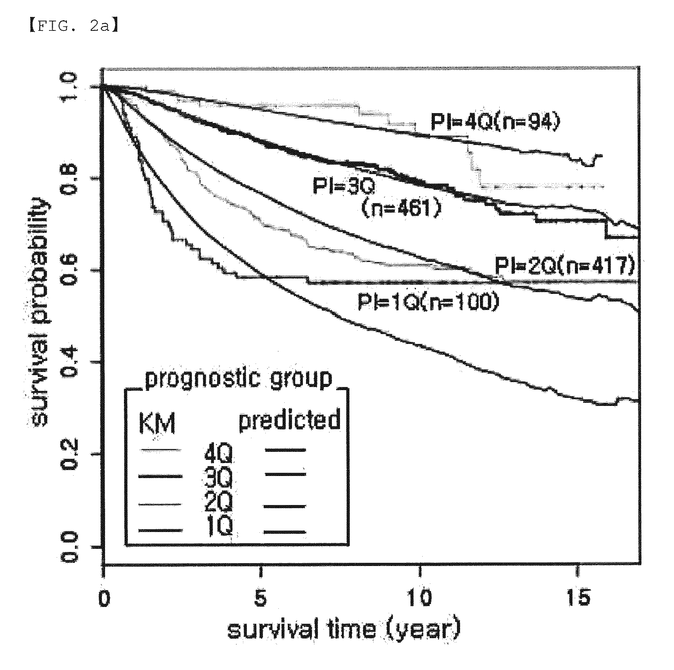

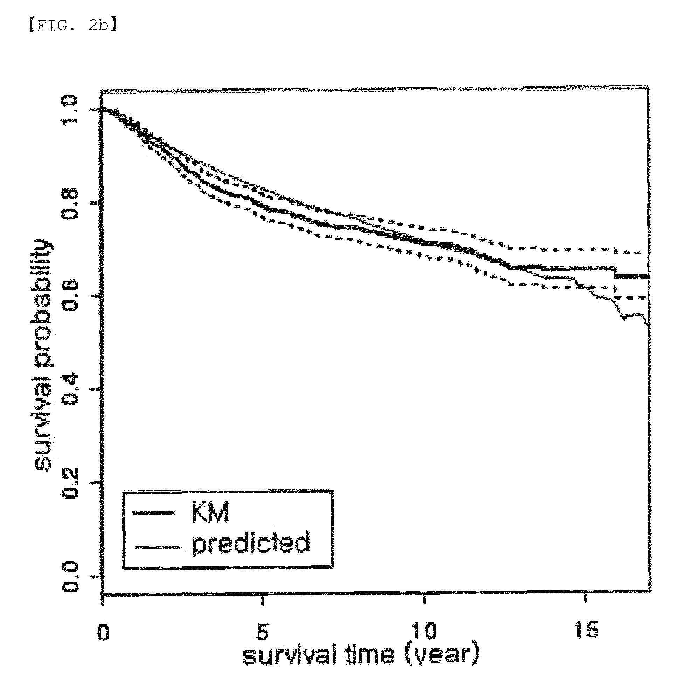

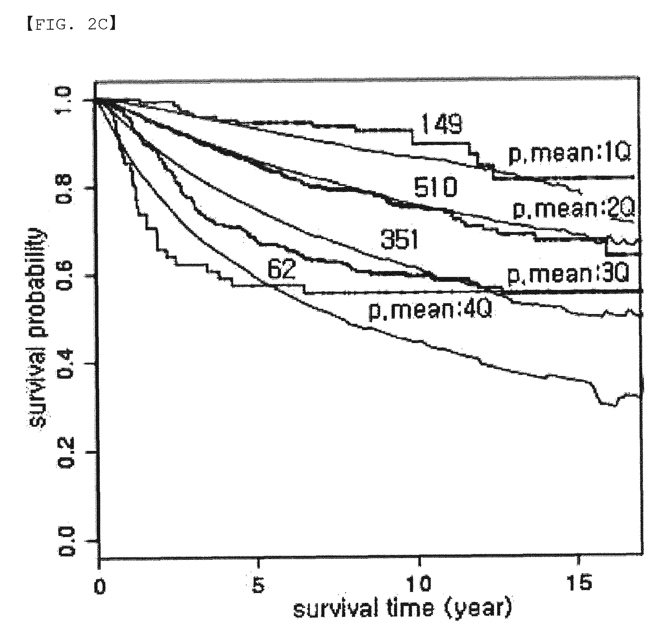

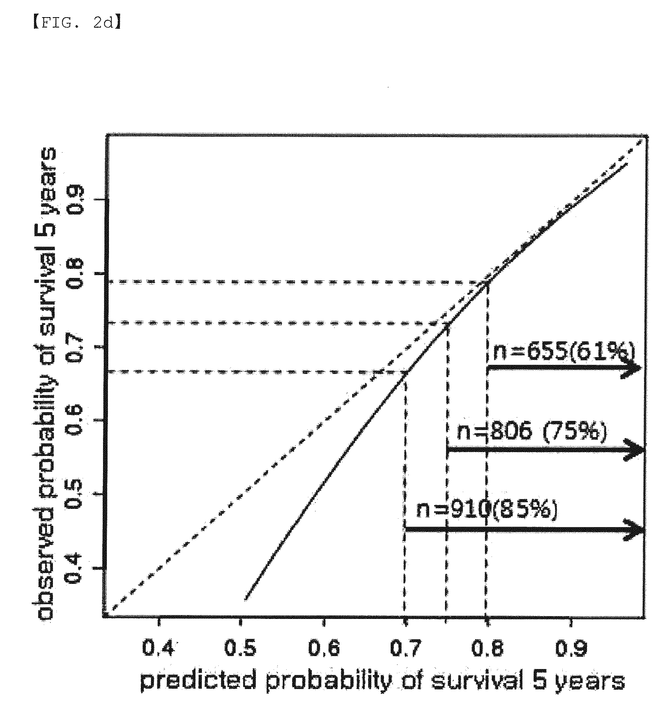

FIG. 2 shows validation results of the prognostic model (frozen samples) in the discovery dataset. FIG. 9a shows that prognostic indexes of all patients using the prognostic model (frozen samples) were divided into four and classified into four prognostic groups, and the separation of the observed survival probability of each prognostic group was verified. The observed survival probability was compared with the predicted survival probability. FIG. 9b shows that observed survival probabilities were compared with predicted survival probabilities using the prognostic models in overall patients. FIG. 9c shows that overall patients were divided into four groups with respect to the most influential p.mean, and then the concurrence between the observed survival probability and the predicted survival probability by the prognostic model (frozen samples) in each group was verified. FIG. 9d shows that the concurrence between the observed survival probability and the predicted survival probability with respect to 5-year survival probability was verified.

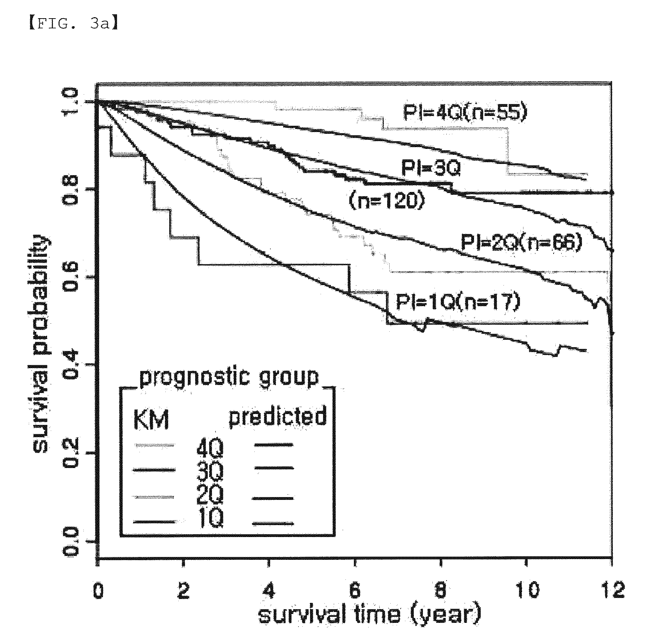

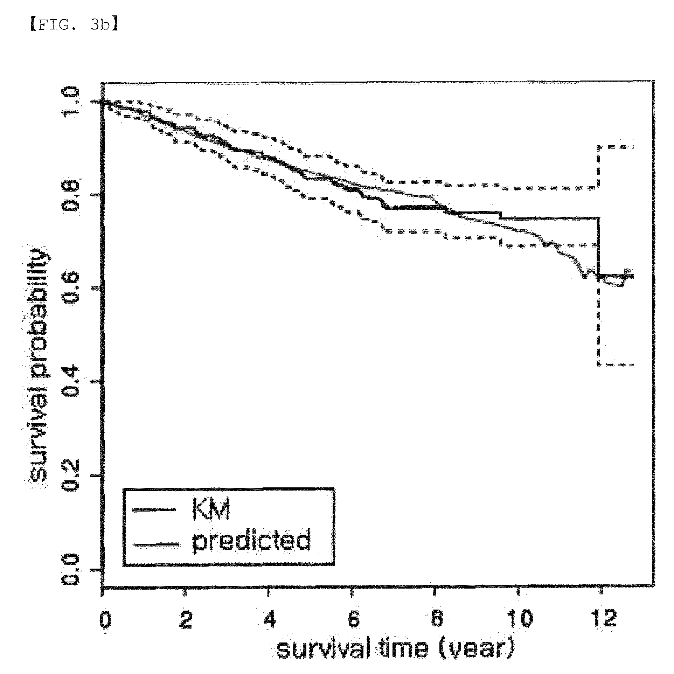

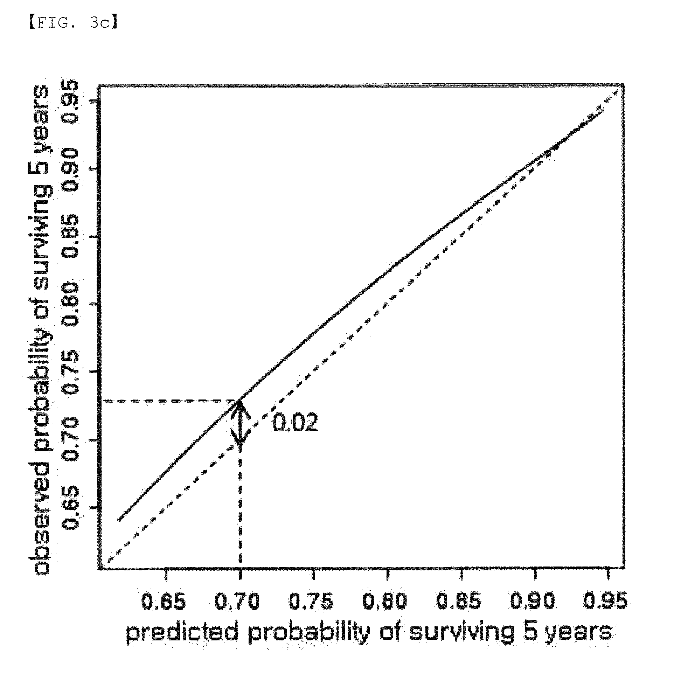

FIG. 3 shows validation results of the prognostic model (frozen samples) in validation set 1. The validation method was the same as that in the discovery dataset. FIG. 10a shows validation results on the determination, and FIG. 10b shows validation results on the calibration of the overall time period of observation. FIG. 10c shows validation results on the calibration of 5-year survival probability.

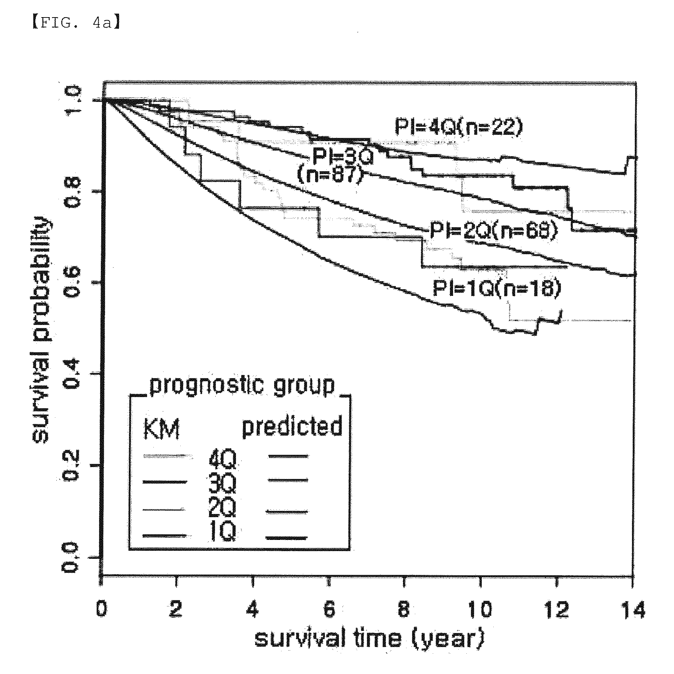

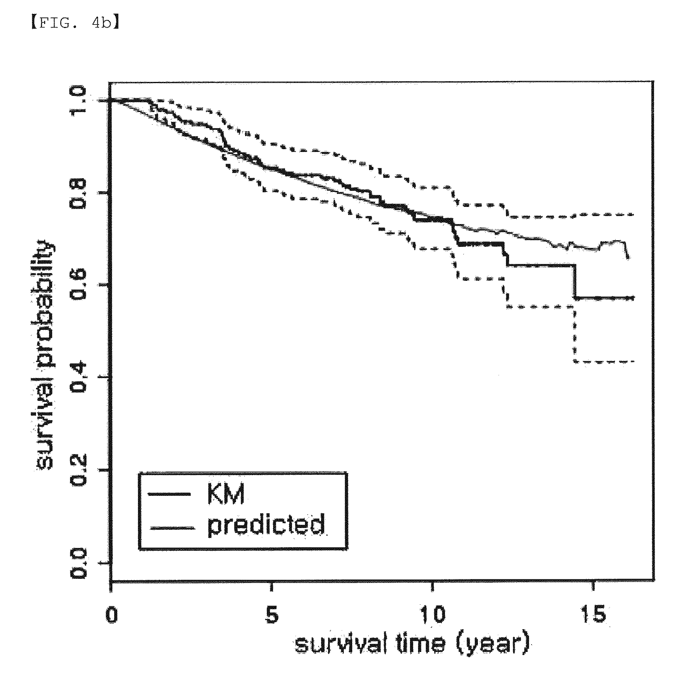

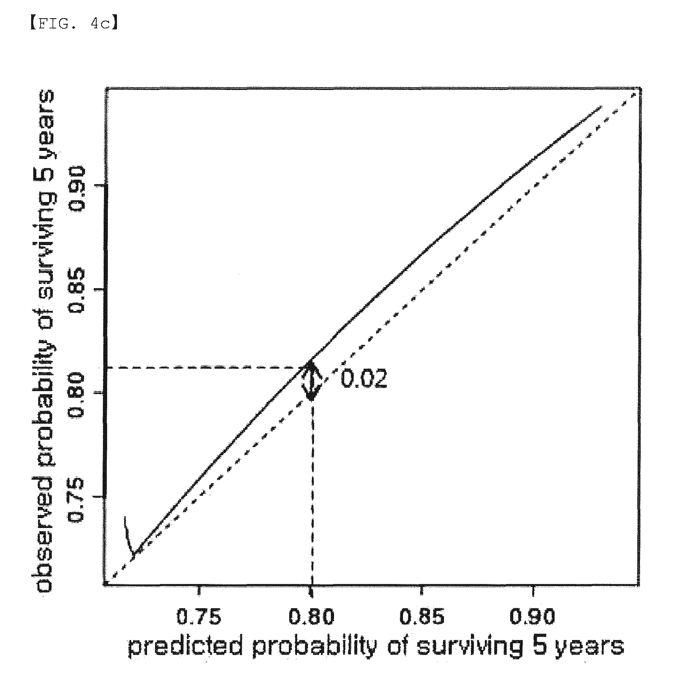

FIG. 4 shows validation results of the prognostic model (frozen samples) in validation set 2. The validation method was the same as that in the discovery dataset. FIG. 11a shows validation results on determination, and FIG. 11b shows validation results on calibration of the overall time period of observation. FIG. 11c shows validation results on the calibration of 5-year survival probability.

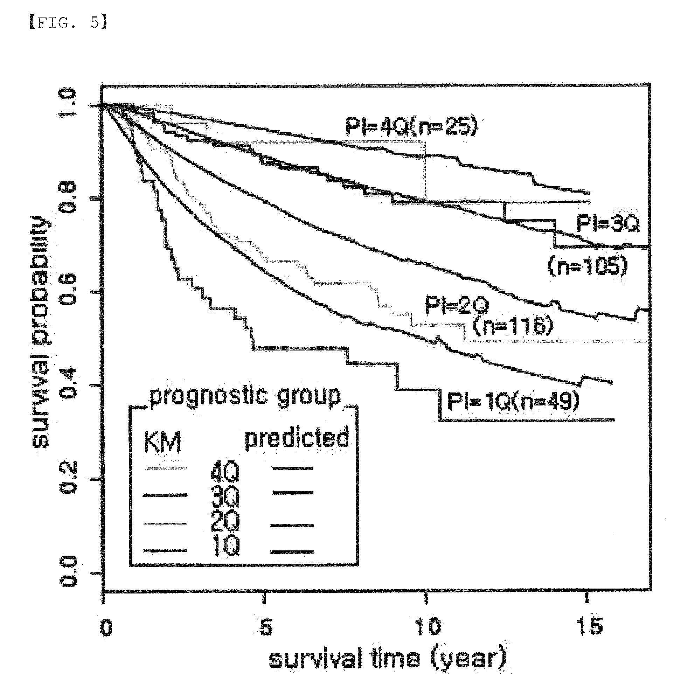

FIG. 5 shows validation results of the prognostic model (frozen samples) in validation set 3. The validation method was the same as that in the discovery dataset.

FIG. 6 shows correlation measurement results between FFPE sample (siemens, vertical axis)/frozen sample (horizontal axis) with respect to selected p-gene, and gene names and correlation values (cor) are shown, respectively.

FIG. 7 shows correlation measurement results between FFPE sample (siemens, vertical axis)/frozen sample (horizontal axis) with respect to selected i-gene, and gene names and correlation values (cor) are shown, respectively.

[Mode for Carrying Out the Invention]

Hereinafter, the present invention will be described in detail with reference to the following examples.

However, the following examples are merely for illustrating the present invention and are not intended to limit the scope of the present invention.

For examples in the present specification, the disclosures of Korean Patent Application Publication No. 10-2012-0079295 and PCT Publication No. WO2012093821A2 are entirely incorporated into the present specification by reference, and the level of the technical field to which the present pertain and the details of the present invention are explained more clearly.

<Methods>

Collection of expression profile in early-stage breast cancer tissue

Expression profiles and clinical information obtained using frozen cancer tissues of early-stage breast cancer patients were collected from open database GEO (http://www.ncbi.nlm.nih.gov/geo). Each of a total nine independent expression profile sets was a relatively big dataset composed of 100 or more samples, and was made in order to conduct researches on prognosis of early-stage breast cancer patients (2, 4, 9, 10, 13, 25, 32, 33). Of these, eight datasets were made using microarray platform, Affymetrix U133A, and the other one dataset was manufactured using Agilent Hu25K. In most cases, important clinical information (age, sex, cancer size, cancer metastasis state, and degree of cancer differentiation) and survival information of patients were collected together. Of the eight datasets made by Affymetrix U133A, six datasets included survival information about distant metastasis (distant metastasis free survival), and the other two datasets included survival information about overall survival. Agilent data included survival information about distant metastasis. Survival analysis on the base of whether distant metastasis occurred or not was performed based on the facts that distant metastasis is the most decisive event in deciding prognosis, the distant metastasis is determined by unique characteristics of cancer, and the largest number of patients had information on the distant metastasis in the collected data. Through comparision of the collected information of all patients, the expression profiles of duplicated 186 cases were removed, and a total of 1,861 unique cases were researched. With respect to seven datasets made by the same platform (Affymetrix U133A), raw files (.CEL) of the expression profiles of corresponding patients were combined together, and then normalized. The normalization was conducted by the rma method (background correction: rma, normalization: quantile, summarization: medianpolish). For the normalization, custom CDF (http://brainarray.mbni.med.umich.edu/Brainarray/) ENTREZG version 13 developed by Manhong Dai et al., was used (34). After the normalization, the expression level of each probe was converted from the one-color expression level into, for example, the two-color expression level by subtracting the mean value of each probe in the discovery dataset. Of the total eight normalized dataset, five datasets were combined together, and then used as the discovery dataset, while two datasets were separately combined, and then used as validation dataset 1, and the other dataset was used as validation dataset 2. Agilent dataset was also used as validation dataset 3.

Prognosis of patients and definition of ER status

In order to identify genes associated with prognosis of patients, the collected patients were classified into a good (favorable) prognostic group and a poor (unfavorable) prognostic group. In general clinical practice, five-year survival or metastasis information is used for such a classification. In other words, the prognosis is poor if metastasis or death occurs within five years, and the prognosis is good if metastasis or death does not occur within five years. The distribution of survival times of metastatic patients was investigated using patient information in the discovery dataset. About 73% or more of the metastatic patients had metastasis within 5 years, and less than 7% of them had metastasis after 10 years. Based on this fact, among patients in the discovery dataset, 217 patients who had metastasis within five years were classified as the "poor prognostic group", while 281 patients who had no metastasis for ten years or longer were classified as the "good prognostic group". As a result of classification, the median survival time of the good prognostic group was 2.4 years, while the median survival time of the poor prognostic group was 12.9 years. Error due to unreliable survival information could be minimized by clearly dividing the poor prognostic group and the good prognostic group. The expression or not of the estrogen receptor (ER) is the most commonly used criterion when breast cancer patients are classified by subtypes. In clinical practice, the breast cancer patients are classified into ER+ and ER-through ER immunohistochemistry (IHC) readout results by pathologists. Considering that about 200 patients had no ER IHC information in the collected discovery dataset, and the determination of ER IHC was made for each of five datasets constituting the discovery dataset, independently, the ER status was determined using the mRNA expression level of ESR1 gene in the expression profile for each patient. For the patients having ER IHC information, region of convergence (ROC) analysis was conducted using the ER IHC information and the ERS1 mRNA expression level. Through a comparison between the ER IHC results and the ERS1 mRNA expression level, the cutoff for ER status was determined at the point where the accuracy was highest (accuracy=0.88). The cases showing higher expression levels than the cutoff were classified as ER+, and the cases showing lower expression levels than the cutoff were classified as ER-. In the discovery dataset, 864 patients were designated as ER+ and 240 patients as ER-.

Selection of prognostic genes

The good prognosis group was defined as ER+, and the poor prognosis group was defined as ER- in the discovery dataset. 275 patients had good prognosis, while 218 patients had poor prognosis. Through significant analysis of microarray (SAM), genes showing a difference in the expression level among the prognostic groups were investigated. By using the q-value of the SAM analysis results, overexpressed genes in the good prognosis group and the poor prognosis group were selected. As a result of combining the selected genes, a total of 302 gene sets that were not duplicated have been made, and a cluster analysis for identifying expression patterns of these genes was conducted using the principal component analysis (PCA) method. Two principal components were selected, and in order to investigate biological functions related to each principal component, GO function analysis was performed for each cluster.

As a result of GO analysis, principal component 1 was shown to concentrate on the proliferation, whereas principal component 2 was shown to concentrate on the immune response. From genes belonging to two principal components involved in the proliferation and the immune response, genes showing the highest expression level between two prognosis groups were selected, respectively. The genes of the respective gene sets are named as p-gene in view of representing expression patterns of the proliferation and i-gene in view of representing expression patterns of the immune response, respectively.

Configuration of prognostic model using parametric survival analysis

Regression analysis in which expression levels of p-gene and i-gene were covariates was performed using the accelerated failure time (AFT) model of parametric survival models. Four p-genes were converted into p.mean by calculating the mean value for each patient, and five i-genes were also converted into i.mean by calculating the mean value of each patient before application. AFT model is specified as:

$$T_i = T_0 \exp(\beta_1 \chi_1 + \beta_2 \chi_2 + \cdots + \beta_q \chi_q) \varepsilon_i \quad (1)$$

wherein, $T_i$ is the survival time of the i-th object; $T_0$ is the baseline survival time; $\chi_i$ is the vector of the covariates (j=1, 2, ..., q); $\beta$ is the coefficient of corresponding covariates; and $\varepsilon$ is the error. In this model, the covariates synergistically influence the baseline survival time, and thus this model is called the accelerated failure time (AFT) model in the industries in which this model is frequently used. The synergistic effect on the survival time, $\Phi = \beta_1\chi_1 + \beta_2\chi_2 + ... + \beta_q\chi_q$ is called the acceleration factor. Where the natural logarithm in Equation (1) is calculated,

$$\log T_i = \log T_0 + \beta_1\chi_1 + \beta_2\chi_2 + \cdots + \beta_q\chi_q + \varepsilon^*(2).$$

Thus, the AFT model has the same form as the general linear regression model. However, since the dependent variable log T does not normally distribute and the survival analysis data always includes censored cases, which are not permitted in the linear regression model, Equation (2) cannot be processed like in the linear regression model. Since the distribution of $\varepsilon^*$ of Equation (2) may vary for different datasets, unlike where the normal distribution is assumed in the linear regression model, the practical statistical processing is inconvenient. In order to overcome this, $\log T_0$ and $\varepsilon^*$ are modified, and expressed as follows:

$$\log T_i = \log T_0 + \beta_1\chi_1 + \beta_2\chi_2 + \cdots + \beta_q\chi_q + \sigma W \quad (3)$$

wherein W follows the distribution of logT, and the variance thereof is fixed as the value of the normalized distribution. Here, $\sigma$ is the constant as the scale parameter, and the value thereof is determined depending on the dataset.

Various candidate prognostic models were fit to Weibull, loglogistic, and lognormal distributions, using AFT model, and then the most appropriate model was selected. For the risk distribution for AFT model, the hazard function, which can be obtained by creating the generation life table of survival information in the discovery dataset, was used. Since the hazard function obtained by the generation life table is shown to have a unimodal form, it was predicted that the Weibull, loglogistic, and lognormal distributions would be well appropriate. The final model was selected in consideration of Akaike's information criterion (AIC) and the R square (R2).

Validation of prognostic model

The performances of the selected model were assessed according to their "calibration" and "discrimination" aspects. "Calibration" is the degree of concurrence between the predicted survival probability produced by the prognostic model and the actually observed survival probability, and "Discrimination" is the ability of the prognostic model to correctly separate the given patient group into different prognostic groups. Herein, the actually observed survival probability refers to the value obtained by Kaplan-Meier method. The AFT-based prognostic model was used to obtain survival probabilities of all time zones for each patient. The survival probability predicted by the model was compared with the survival probability by Kaplan-Meier method. In order to obtain the predicted survival probabilities for the overall time period like in Kaplan-Meier, survival probability curves of all patients were obtained by calculating the mean survival probability for each time zone from 0 to 25 years by the 0.1 year unit. Together with the comparison of survival probability for the overall survival time, the 5-year survival probability was also compared. The 5-year predicted survival probability of patients produced by using the prognostic model in the given dataset was compared with, as the observed value, the 5-year survival probability calculated by using Hare which is the hazard regression analysis.

For "discrimination", the prognostic indexes of all patients in the given dataset were divided into four sections, and survival probabilities of patients for the respective sections were compared by the KM graph. The prognostic index is the dependent variable in the survival model. The more clearly the KM graphs of the four prognostic groups are separated, the better the discrimination power of the model.

Both "calibration" and "determination" for the discovery dataset and three independent validation datasets were investigated.

Important R packages used in statistical analysis are as below:

affy: pre-processing of .CEL files using rma algorithm
samr: identifying genes showing the difference in the expression level between prognostic groups
GOstats: investigating function associated with selected gene set
KMsurv: creating a life table using survival data of the discovery dataset
rma: estimating coefficients of prognostic model using AFT model and conducting calibration on the model.

Selection of gene sets applicable to FFPE sample

RNA extracted from formalin-fixed paraffin embedded (FFPE) tissues and samples has not been accepted in the expression analysis due to many procedures causing the obstruction to RNA stability, such as immobilization causing cross-linking between tissues in the treatment process of tissues or samples. Herein, in order to develop a method for breast cancer prognosis prediction, suitable for FFPE samples, based on the actual breast cancer treatment procedure, gene sets were selected by placing a higher priority on genes having higher interqurtile ranges (IQR) and genes having higher mean expression levels in the order of higher degree of contribution, from p-genes (proliferation-related gene set) representing the expression pattern of proliferation and i-genes (immune response-related gene set) representing the expression pattern of the immune response, which were obtained according to the principal component analysis and function analysis with respect to the principal component analysis (GO function analysis or GO analysis).

As used herein, several genes having the same pattern in p-gene and i-gene were selected since the microarray data has a limitation in measuring accurate expression levels and the mean expression level for several genes rather than the expression level of one gene representing the pattern can represent the actual expression pattern better.

Measurement of gene correlation between FFPE sample and Frozen sample and selection of genes

27 types of FFPE and frozen samples collected from the same patients were secured, and RNA was extracted from the FFPE or frozen samples by the RNA extraction method. The expression levels of 32 types of genes selected were measured by using the extracted RNA as a template.

The gene expression levels need to be normalized due to the difference thereof between individuals. Therefore, the expression levels of five genes selected as a normalization gene, that is, of C-terminal-binding protein 1 (CTBP1), TATA-binding protein (TBP), hydroxymethylbilane synthase (HMBS), cullin 1 (CUL1), and ubiquilin-1 (UBQLN1), were normalized, and then the gene expression correlation between FFPE sample and frozen sample was measured.

Based on the correlation measurement results and the expression level results for each gene, the genes that have a high correlation rate and various gene expression distributions between samples were finally selected as highly reliable genes for early-stage breast cancer prognosis prediction.

<Results>

Selection of prognostic genes for prognostic model

Five independent breast cancer datasets composed of expression profiles of early-stage breast cancer tissue were pooled into a discovery dataset of 1,104 samples. All patients did not receive chemotherapy, while most of them have little metastasis to axillary lymph nodes (N0 or N-), or have early-stage breast cancer (1st stage or 2nd stage). Among them, 1072 patients with information about distant metastasis were subjected to statistical analysis. In order to find genes associated with prognosis, expression profiles of the good prognostic group (having no metastasis for more than 10 years) and the poor prognostic group (having metastasis within 5 years) were compared. 182 genes showing high expression levels were selected from the good prognostic group, and 120 genes showing high expression levels were selected from the poor prognostic group (results not shown, FDR<0.001).

Principal component analysis was performed on the expression levels of the selected 302 genes. GO function analysis was performed on principal component 1 and principal component 2. Principal component 1 was definitely associated with the proliferation, while principal component 2 was strongly associated with the immune response. Based on this result, genes belonging to principal components 1 and 2 were selected, respective, and thus the prognostic model was allowed to reflect the two expression patterns.

Nine genes that were not only associated with the prognosis but also had the largest expression difference among the groups were selected. The gene selected from principal component 1 representing the proliferation is named p-gene, and the gene selected from principal 2 representing the immune response is named i-gene.

Comparison ER+ Breast Cancer and ER- Breast Cancer

It is known that the expression or not of the estrogen receptor (ER) is closely associated with the occurrence and development of breast cancer. Two functions shown in the genes selected in association with prognosis, that is, proliferation and immune response are interestingly noticed in the mechanism of cancer. ER- breast cancer was compared with ER+ breast cancer using the selected 16 genes (p-genes and i-genes). In order to display the intensity of each function, the p-genes and i-genes were stratified into three stages (p1, p2, p3 or i1, i2, i3) according to the expression level. Here, p1 was a p-gene group showing the lowest expression level, and was assumed to make the slowest proliferation; p3 was a p-gene group showing the highest expression level, and was assumed to make the highest proliferation; and p2 was a p-gene group showing a moderate expression level, and was assumed to make a moderate level of proliferation. As used herein, i1 was an i-gene group showing the lowest expression level, and was assumed to make the weakest immune response; i3 was an i-gene group showing the highest expression level, and was assumed to make the strongest immune response; and i2 was considered to show a moderate expression level, and was assumed to do a moderate level of activity.

1,072 cases in the discovery dataset were classified according to the expression levels of p-gene and i-gene, and the intensity of each function according to the ER status was investigated. The proportion of p3 type indicating very active proliferation was higher in ER- breast cancer than in ER+ breast cancer. About 62% of ER- breast cancer cases showed very high p-gene expression levels (p3), while only 18% of ER+ breast cancer cases showed high p-gene expression levels, which supported the previously reported fact that ER- breast cancer tends to be more aggressive than ER+ breast cancer. About 35% of ER+ breast cancer cases showed weak p-genes (p1), while the proportion of p1 was only 9% in ER- breast cancer cases. The active immune response function is another characteristic of ER- breast cancer, and 38% or more of ER- breast cancer cases showed very high i-gene expression levels (i3). Whereas, only 21% of ER+ breast cancer cases showed high i-gene expression levels. It was observed that more active proliferation led to more active immune response in both ER+ and ER-, while ER- breast cancer showed a much more active immune response.

Besides, it was observed that the differentiation grade of the breast cancer was also closely related with the proliferation. Generally, poorly differentiated breast cancer (G3) showed a fast proliferation, whereas well-differentiated breast cancer (G1) showed a slow proliferation. It was observed that the prognosis of patients was also correlated with the proliferation. It was observed that more poor prognostic patients having metastasis within five years were concentrated in the group with a fast proliferation.

In summary, it was found that ER- breast cancer was more active than ER+ breast cancer in view of both the proliferation and the immune response, and it was supposed that the expression level of ER influences the mechanisms of occurrence and development of breast cancer.

Establishment of prognostic model

The AFT prognostic model of early-stage breast cancer patient metastasis was created using survival information of the discovery dataset and the selected p-genes and i-genes. The generation life table on a yearly basis was created using the survival information of the discovery dataset, and the degree of hazard was roughly calculated.

Since the probability of death obtained by the generation life table is shown to have a unimodal form, it was predicted that the Weibull, loglogistic, and lognormal distributions would be well appropriate. The covariates to be included in the prognostic model are p.mean and i.mean. p.mean is the mean in the p-genes, and i.mean is the mean in the i-genes.

As a result of applying three models to the Weibull, loglogistic and lognormal distributions, the lognormal distribution was most appropriate. The final model following the lognormal distribution was selected using Akaikes information criterion (AIC).

$$\log(T) = -0.689 \times p.mean + 0.274 \times i.mean + 3.219$$

According to the above estimated model, the p.mean, that is, the proliferation has a negative correlation (-0.689, p value = $2.47 \times e^{-17}$) with the survival time (T), and thus, more active proliferation shortens the survival time. On the contrary, the i.mean has a positive correlation (0.274, p value = $3.69 \times e^{-11}$) with the survival time, which indicates that a more active immune response lengthens the survival time. It could be concluded from the above estimated factors that proliferation plays a decisive role in the breast cancer prognosis, and more active proliferation leads to poor prognosis, while the immune response acts as a defense mechanism against fast proliferation.

Validation of prognostic model

The performances of the prognostic model, which was created using expression profiles for 1,072 early-stage breast cancer patients in the discovery dataset, were validated according to "calibration" and "discrimination" aspects. Here, the "calibration" is the degree of concurrence between the predicted survival probability produced through the model and the actually observed survival probability. The actually observed survival probability refers to the survival probability obtained using the Kaplan-Meier method. In addition, the "discrimination" is the ability of the model to correctly separate the patients into different prognostic groups. Validations for the two performances were performed on the discovery dataset developing the model and three independent validation datasets.

In the discovery dataset developing the prognostic model, the prognostic indexes (PI) were divided into four and classified into four prognostic groups. The survival probabilities observed in the four prognostic groups classified by the prognostic index were compared using the KM graph. As a result, it could be seen that four prognostic groups were very well classified, and the predicted survival probability corresponded well with the observed survival probability for each prognostic group.

The KM survival probability and the predicted survival probability produced by the prognostic model were compared with each other using the graph. The survival probabilities for all time zones of all patients were obtained in the prognostic model, and thus, in order to obtain the survival probability curve for the overall time period like the survival curve of KM, survival probability curves were drawn using the mean survival probability for each time zone (from 0 to 25 year, 0.1-year interval) of each patient. The predicted survival probability was slightly higher than the KM survival probability, but they are similar as a whole. In addition to the comparison of survival probability for the overall survival time, the comparision of 5-year survival probability was also conducted. The 5-year survival probability predicted by the model was similar to the actually observed 5-year survival probability. Particularly, as the predicted survival probability was higher, the concurrence between the predicted probability and the observed survival probability was stronger.

For more objective validation, the prognostic model was validated using three independent validation datasets. The first validation dataset was obtained by combining two datasets generated by Affymetrix U133A platform. The second validation dataset was the dataset generated by Affymetric U133A platform, and all were ER+ patients taking tamoxifen for five years. The third validation dataset was the dataset that was used to identify and validate 70 prognostic genes (currently, commercialized as MammaPrint) and generated by Agilent Hu25K platform. Validation datasets 1 and 2 were generated by the same platform as in the discovery dataset, Affymetrix U133A, and the expression levels thereof were normalized together with the discovery dataset. Validation datasets 1 and 2 were assessed in terms of calibration and discrimination aspects, while validation datasets 3 was assessed in terms of only the discrimination aspect due to the normalization of expression levels.

Selection of gene sets applicable to FFPE sample

32 types of genes were selected by placing a higher priority on genes having higher interqurtile ranges (IQR) and genes having higher mean expression levels in the order of higher degree of contribution, from 182 genes showing high expression levels in the good prognostic group and 120 genes showing high expression levels in the poor prognostic group in the discovery dataset of 1,104 samples.

Measurement of gene correlation FFPE sample and Frozen sample and selection of genes

For the measurement of correlation between FFPE sample and frozen sample, samples that secure both FFPE sample and frozen sample from patients or their cancer tissues are needed. With respect to 27 pairs of FFPE and frozen samples thus obtained, the expression levels of 32 types of the selected genes and the correlation between FFPE and frozen samples were measured. Based on the measurement results, genes that have a high correlation rate and various gene expression distributions among samples were found 12 types in p-genes and 15 types in i-genes, which were selected as highly reliable genes for early-stage breast cancer prognosis prediction.

Among the selected genes above, 9 types of p-genes and 6 types of i-genes were selected as genes to be included in the kit for prognosis prediction.

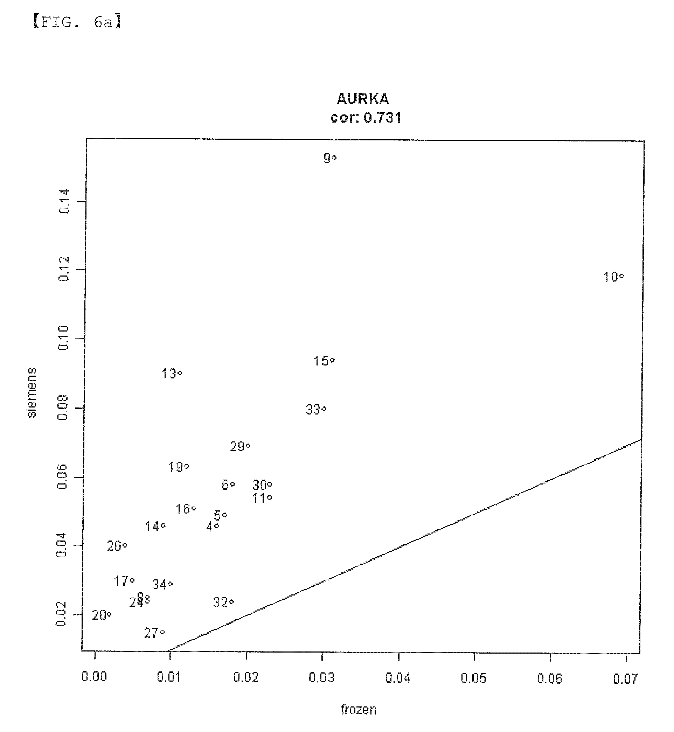

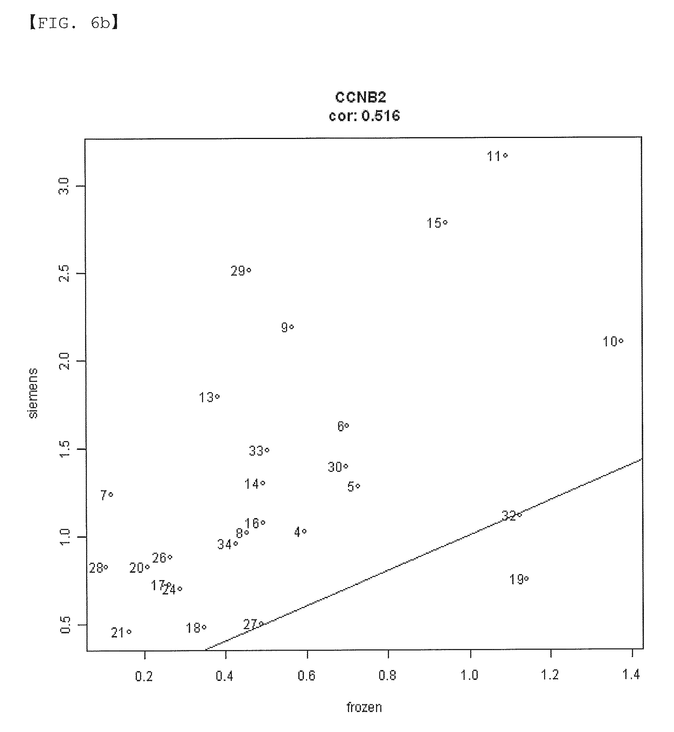

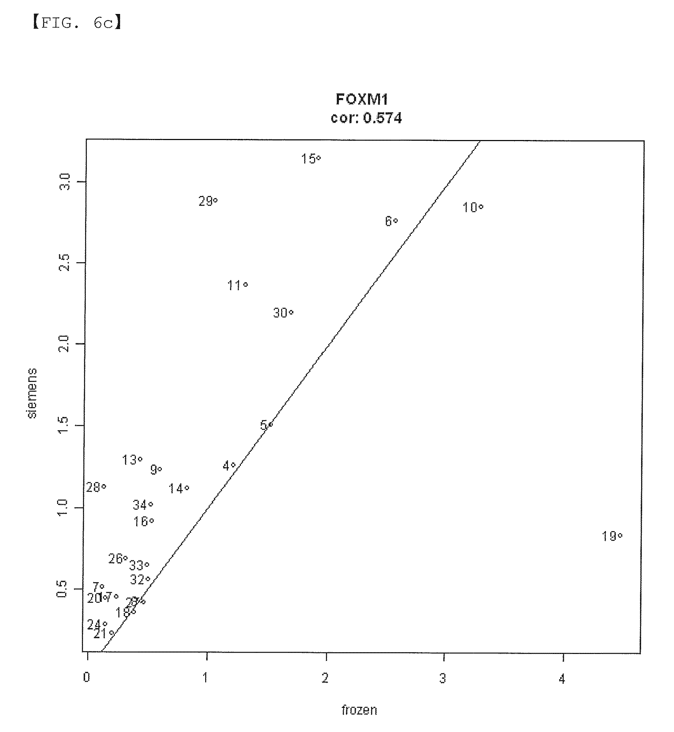

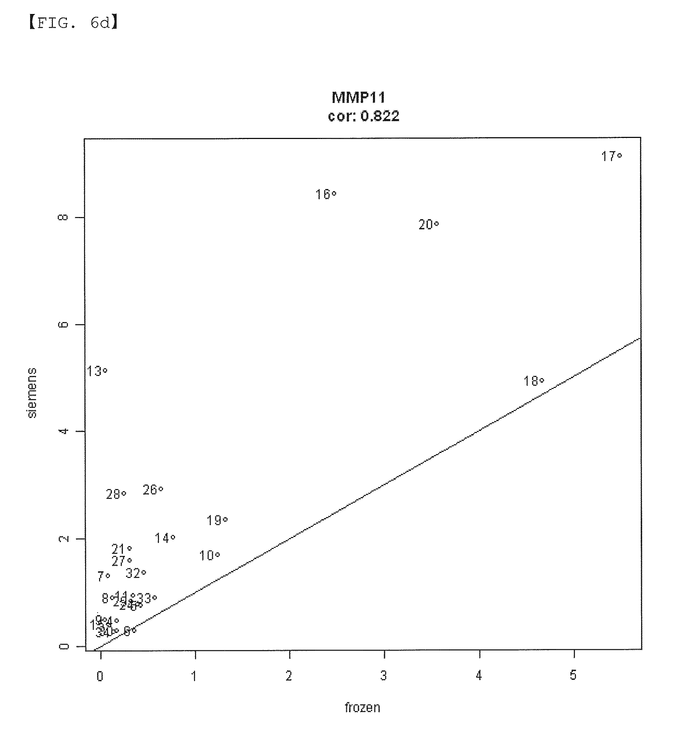

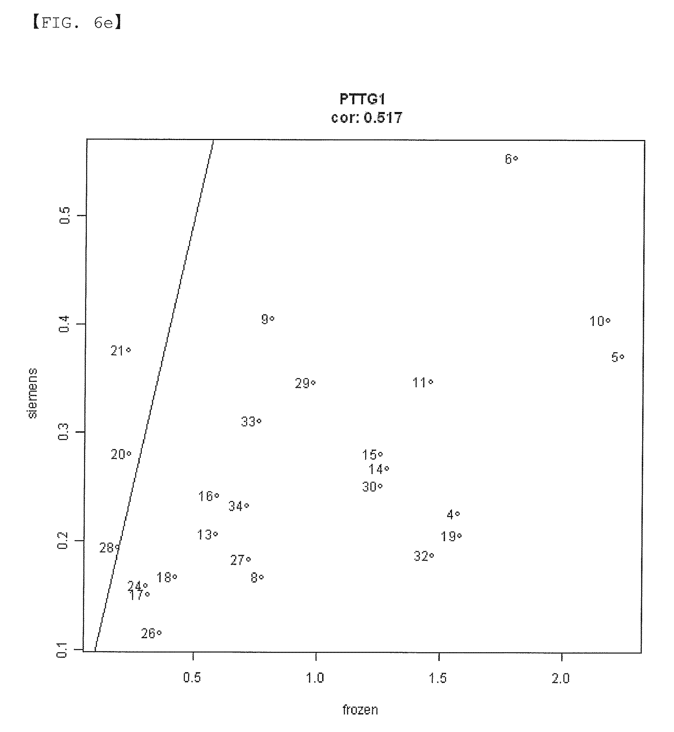

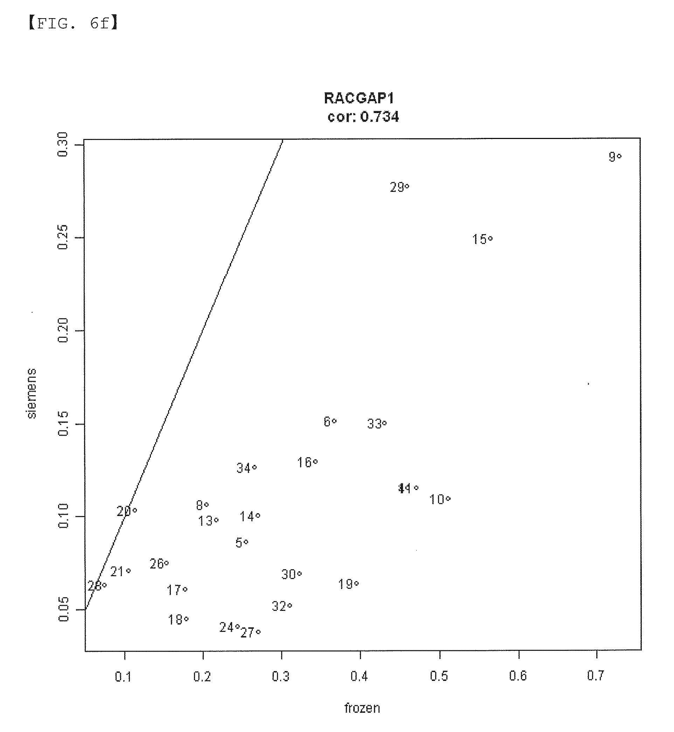

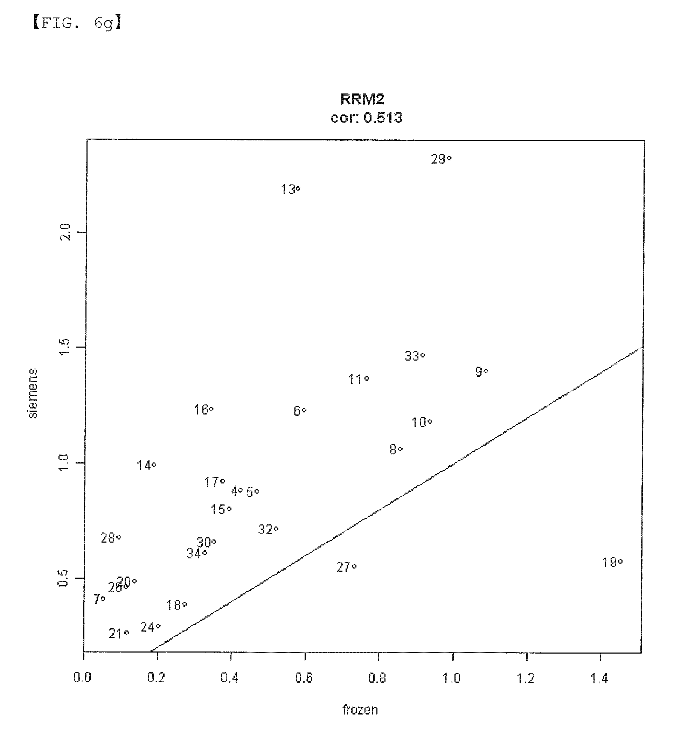

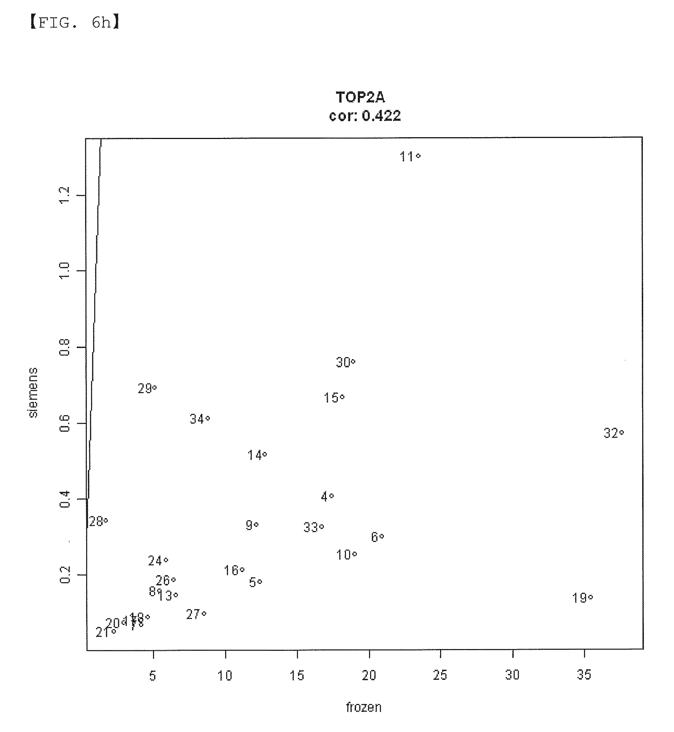

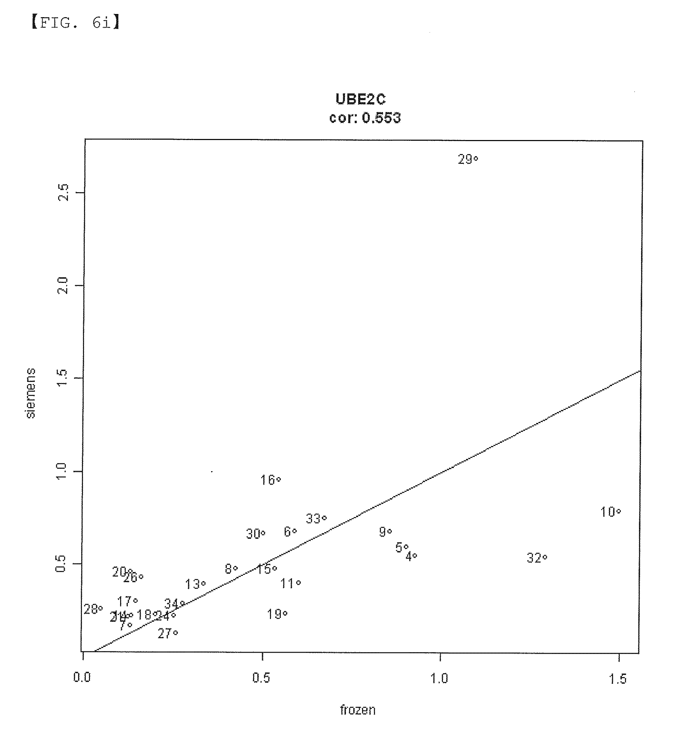

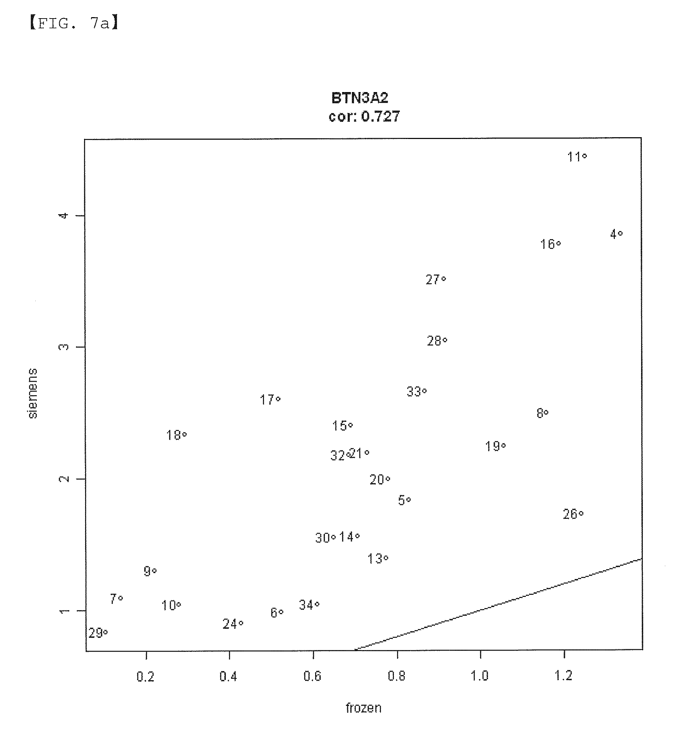

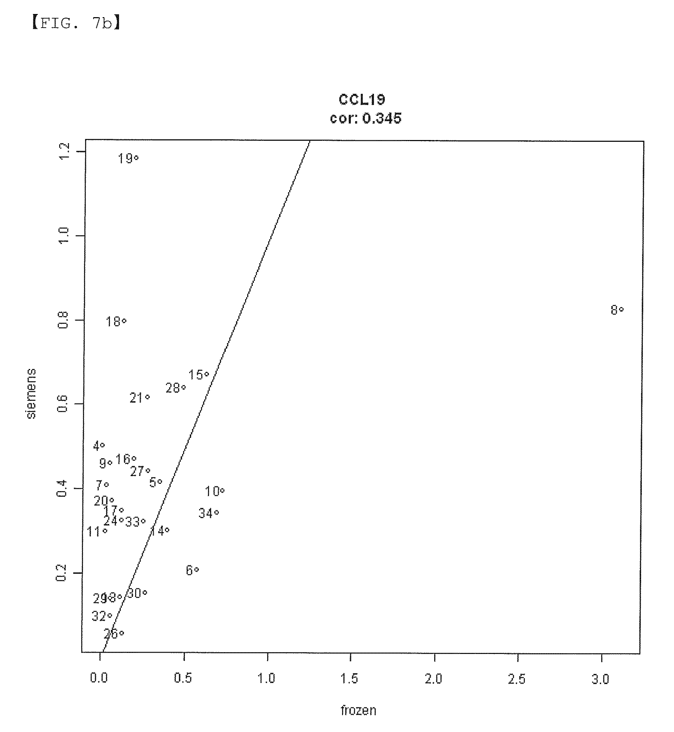

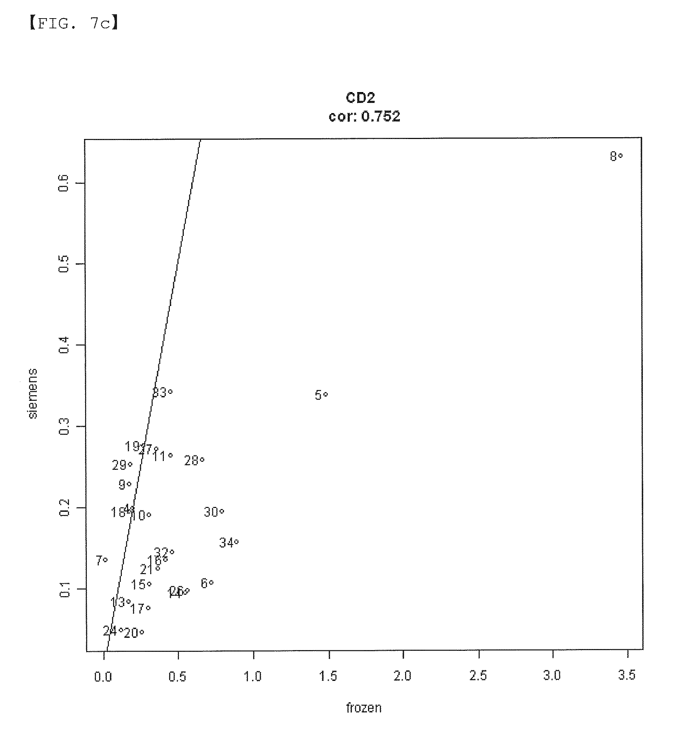

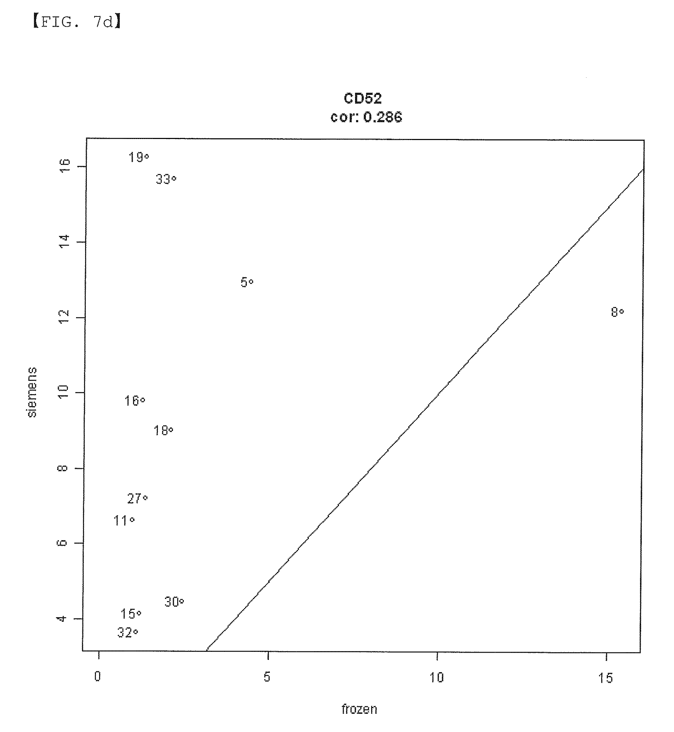

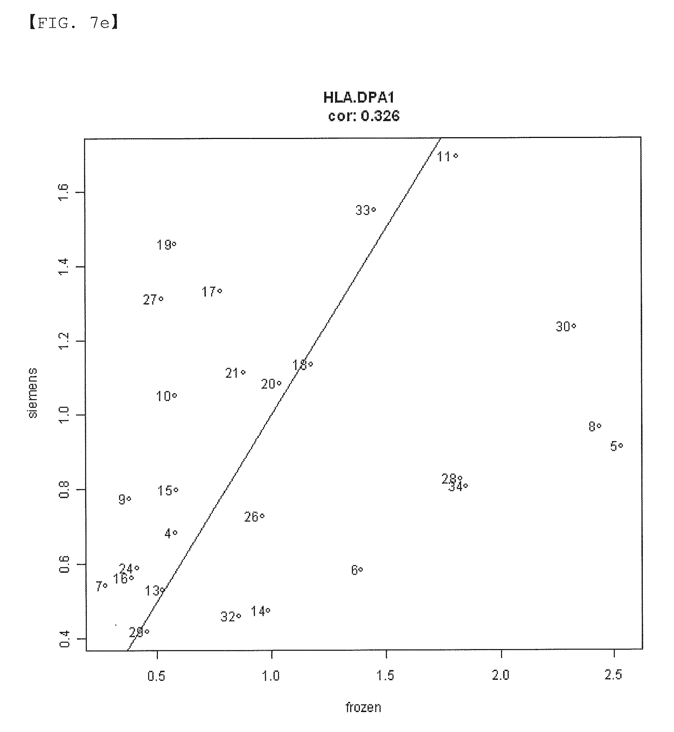

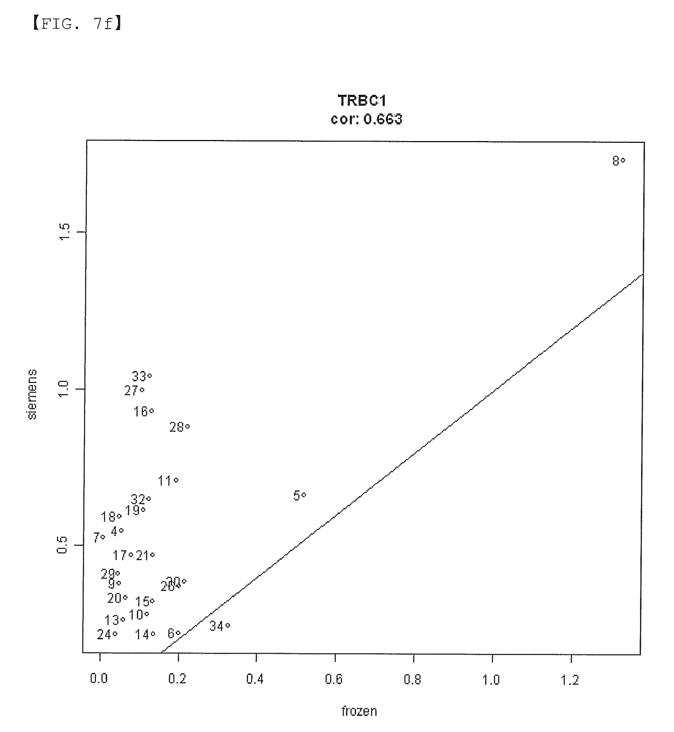

The correlation measurement results for the respective genes are shown in FIGs. 6 and 7. In each drawing, the solid line represents the equivalent value of the horizontal axis and the vertical axis (slope: 1, that is, the line that connects values of which the horizontal axis value is identical to the vertical axis value).

Meanwhile, among the respective genes, the expression levels of TRBC1, BTN3A2, and HLA-DPA1 were significantly different between the good prognostic group and the poor prognostic group. Through the above analysis, it could be concluded that the expression of TRBC1, BTN3A2, and HLA-DPA1 was significantly increased in the good prognostic group, verifying that their increased expression (overexpression) indicated good breast cancer prognosis.

[Industrial Applicability]

As set forth above, the present invention provides a genetic marker for early-stage breast cancer prognosis prediction and diagnosis. The genetic marker of the present invention can enable the prediction or diagnosis of the prognosis of a breast cancer patient, and thus can be favorably used to present clues for the future direction of breast cancer treatment, including the determination on whether anticancer treatment is needed.

**Claims**

1.   A method for predicting the prognosis of breast cancer, the method comprising:

     (a) isolating mRNA from a sample;

(b) measuring the mRNA expression level of at least one gene selected from the group consisting of TRBC1 (T cell receptor beta constant 1), BTN3A2 (butyrophilin, subfamily 3, member A2), and HLA-DPA1 (major histocompatibility complex, class II, DP alpha 1);

(c) normalizing the mRNA expression level of the gene; and

(d) determining the overexpression of the gene as indicating good prognosis.

2. The method of claim 1, wherein the measuring of the expression level is conducted through PCR amplification of a target gene.

3. The method of claim 1, wherein the sample is a formalin-fixed paraffin-embedded (FFPE) sample of a tissue containing cancer cells of a patient.

4. The method of claim 1, wherein the normalizing is conducted by calculating the ratio of the mean expression level compared with at least one standard gene selected from the group consisting of CTBP1 (C-terminal-binding protein 1), TBP (TATA-binding protein), HMBS (hydroxymethylbilane synthase), CUL1 (cullin 1), and UBQLN1 (ubiquilin-1).

5. A composition for predicting or diagnosing the prognosis of breast cancer, the composition comprising a primer pair as an active ingredient, wherein the primer pair is for at least one gene selected from the group consisting of TRBC1 (T cell receptor beta constant 1), BTN3A2 (butyrophilin, subfamily 3, member A2), and HLA-DPA1 (major histocompatibility complex, class II, DP alpha 1), and wherein the primer pair is capable of amplifying a target gene through PCR amplification.

6. A kit for predicting or diagnosing the prognosis of breast cancer, the kit comprising the composition of claim 5.

7. A method for calculating a predictive value of the prognosis of breast cancer to provide information necessary for predicting or diagnosing the prognosis of a breast cancer patient, the method comprising:

(a) isolating mRNA from a sample;

(b) measuring the mRNA expression level of at least one gene selected from the group consisting of TRBC1 (T cell receptor beta constant 1), BTN3A2 (butyrophilin, subfamily 3, member A2), and HLA-DPA1 (major histocompatibility complex, class II, DP alpha 1);

(c) normalizing the mRNA expression level of the gene;

(d) inserting the normalized value into a predetermined calculation formula to calculate a numerical value; and

(e) determining the prognosis of breast cancer as being good or poor depending on the numerical value.

8. The method of claim 7, wherein the measuring of the expression level is conducted through PCR amplification of a target gene.

9. The method of claim 7, wherein the sample is a formalin-fixed paraffin-embedded (FFPE) sample of a tissue containing cancer cells of a patient.

10. The method of claim 7, wherein the normalizing is conducted by calculating the ratio of the mean expression level compared with at least one standard gene selected from the group consisting of CTBP1 (C-terminal-binding protein 1), TBP (TATA-binding protein), HMBS (hydroxymethylbilane synthase), CUL1 (cullin 1), and UBQLN1 (ubiquilin-1).

11. A method for predicting or diagnosing the prognosis of breast cancer, the method comprising a step of using a primer pair to measuring a mRNA expression level of a selected gene from a sample of a breast cancer patient, wherein the primer pair is for at least one gene selected from the group consisting of TRBC1 (T cell receptor beta constant 1), BTN3A2 (butyrophilin, subfamily 3, member A2), and HLA-DPA1 (major histocompatibility complex, class II, DP alpha 1), and wherein the primer pair is capable of amplifying a target gene through PCR amplification,

12. A primer pair for at least one gene selected from the group consisting of TRBC1 (T cell receptor beta constant 1), BTN3A2 (butyrophilin, subfamily 3, member A2), and HLA-DPA1 (major histocompatibility complex, class II, DP alpha 1), wherein the primer pair is capable of amplifying a target gene through PCR amplification.

13. Use of a primer pair for preparing an agent for predicting the prognosis of breast cancer, wherein the primer pair is for at least one gene selected from the group consisting of TRBC1 (T cell receptor beta constant 1), BTN3A2 (butyrophilin, subfamily 3, member A2), and HLA-DPA1 (major histocompatibility complex, class II, DP alpha 1), and wherein the primer pair is capable of amplifying a target gene through PCR amplification.

【FIG. 1a】

【FIG. 1b】

1,072 cases
· Lymph node negative
· No chemotherapy

Define prognostic groups
Good prognosis: > 10 yrs no metastasis
Poor prognosis: < 5 yrs mutastasis occurrence

Good prognosis: 275 cases
Poor prognosis: 218 cases

Identify genes exhibiting significant difference in
expression level among prognostic groups (SAM)

Good.up: 120 genes
Poor.up: 182 genes
(FDR<0.001)

Principal component analysis (PCA)

GO function analysis (by PC)

Principal component 1
(PC1): Proliferation

Principal component 2
(PC2): Immune response

Condition for prognostic gene selection
· Principal component analysis result
· Quartile range
· Mean expression level

Prognostic genes
Proliferation: 4 genes
Immune response: 5 genes

Development of prognosis prediction
model (AFT model)

【FIG. 2a】

【FIG. 2b】

【FIG. 2C】

【FIG. 2d】

【FIG. 3a】

【FIG. 3b】

【FIG. 3c】

【FIG. 4a】

【FIG. 4b】

【FIG. 4c】

【FIG. 5】

【FIG. 6a】

【FIG. 6b】

CCNB2
cor: 0.516

【FIG. 6c】

FOXM1
cor: 0.574

【FIG. 6d】

【FIG. 6e】

**PTTG1**
**cor: 0.517**

【FIG. 6f】

RACGAP1
cor: 0.734

【FIG. 6g】

【FIG. 6h】

TOP2A
cor: 0.422

【FIG. 6i】

UBE2C
cor: 0.553

【FIG. 7a】

【FIG. 7b】

CCL19
cor: 0.345

【FIG. 7c】

【FIG. 7d】

**CD52**
**cor: 0.286**

【FIG. 7e】

HLA.DPA1
cor: 0.326

【FIG. 7f】

TRBC1
cor: 0.663

## INTERNATIONAL SEARCH REPORT

| | International application No. |
|---|---|
| | **PCT/KR2014/003384** |

| A. | CLASSIFICATION OF SUBJECT MATTER |
|---|---|

*G01N 33/574(2006.01)i, C12Q 1/68(2006.01)i, G01N 33/53(2006.01)i*

According to International Patent Classification (IPC) or to both national classification and IPC

| B. | FIELDS SEARCHED |
|---|---|

Minimum documentation searched (classification system followed by classification symbols)

G01N 33/574; C40B 40/06; C40B 40/08; C12N 15/12; G06F 19/10; C12Q 1/68; G01N 33/53

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: breast cancer, gene marker, TRBC1, BTN3A2, HLA-DPA1

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | US 2008-0206769 A1 (LAU, Kit et al.) 28 August 2008<br>See abstract; claims 1-3, 7, 10-11, 13 and 17. | 1-4,7-10 |
| A | | 5-6,11-13 |
| X | KR 10-2012-0079295 A (GENCURIX INC.) 12 July 2012<br>See abstract; claim 1; table 2. | 5-6,11-13 |
| Y | | 1-4,7-10 |
| Y | KR 10-2009-0088757 A (SEOUL NATIONAL UNIVERSITY INDUSTRY FOUNDATION) 20 August 2009<br>See abstract; claims 1, 12 and 16-17. | 4,10 |
| A | LE PAGE, CECILE et al., "BTN3A2 Expression in Epithelial Ovarian Cancer Is Associated with Higher Tumor Infiltrating T Cells and a Better Prognosis", PLOS ONE, 2012, vol. 7, no. 6, e38541.<br>See the entire document. | 1-13 |
| A | US 2010-0035240 A1 (JIANG, Wen Guo) 11 February 2010<br>See abstract; claims 1, 5 and 9. | 1-13 |
| A | KR 10-2011-0110031 A (SNU R&DB FOUNDATION) 06 October 2011<br>See abstract; claim 1. | 1-13 |

| ☐ Further documents are listed in the continuation of Box C. | ☒ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 28 JULY 2014 (28.07.2014) | **28 JULY 2014 (28.07.2014)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

**EP 2 988 131 A1**

| INTERNATIONAL SEARCH REPORT | | International application No. |
|---|---|---|
| Information on patent family members | | PCT/KR2014/003384 |

| Patent document cited in search report | Publication date | Patent family member | Publication date |
|---|---|---|---|
| US 2008-0206769 A1 | 28/08/2008 | CA 2674907 A1 | 07/08/2008 |
| | | EP 2129789 A2 | 09/12/2009 |
| | | EP 2129789 A4 | 24/03/2010 |
| | | EP 2520668 A1 | 07/11/2012 |
| | | JP 2010-516292 A | 20/05/2010 |
| | | US 2010-0323921 A1 | 23/12/2010 |
| | | US 7695915 B2 | 13/04/2010 |
| | | WO 2008-094678 A2 | 07/08/2008 |
| | | WO 2008-094678 A3 | 20/11/2008 |
| KR 10-2012-0079295 A | 12/07/2012 | KR 10-1287600 B1 | 18/07/2013 |
| | | KR 10-2013-0023312 A | 07/03/2013 |
| | | US 2013-0344482 A1 | 26/12/2013 |
| | | WO 2012-093821 A2 | 12/07/2012 |
| | | WO 2012-093821 A3 | 24/01/2013 |
| KR 10-2009-0088757 A | 20/08/2009 | NONE | |
| US 2010-0035240 A1 | 11/02/2010 | AT 454474 T | 15/01/2010 |
| | | AU 2006-271113 A1 | 16/02/2006 |
| | | AU 271113 B2 | 09/12/2010 |
| | | CA 2576113 A1 | 16/02/2006 |
| | | CN 102443627 A | 09/05/2012 |
| | | DE 602005018789 D1 | 26/05/2011 |
| | | DK 1781814 T3 | 03/05/2010 |
| | | EP 1781814 A1 | 09/05/2007 |
| | | EP 1781814 B1 | 06/01/2010 |
| | | EP 1781814 B3 | 31/08/2011 |
| | | ES 2339132 T3 | 17/05/2010 |
| | | GB 0701676 D | 14/03/2007 |
| | | GB 2430741 A | 04/04/2007 |
| | | HK 1103422 A1 | 03/09/2010 |
| | | JP 2008-508895 A | 27/03/2008 |
| | | JP 2012-024095 A | 09/02/2012 |
| | | MX 2007001640 A | 25/07/2007 |
| | | PT 1781814 E | 12/04/2010 |
| | | WO 2006-016110 A1 | 16/02/2006 |
| | | WO 2006-016110 A8 | 24/08/2006 |
| KR 10-2011-0110031 A | 06/10/2011 | WO 2011-122857 A2 | 06/10/2011 |
| | | WO 2011-122857 A3 | 23/08/2012 |

Form PCT/ISA/210 (patent family annex) (July 2009)

44

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130043160
- KR 1020120079295
- WO 2012093821A2 A

**Non-patent literature cited in the description**

- **CHANG, H.Y. et al.** Gene expression signature of fibroblast serum response predicts human cancer progression: similarities between tumors and wounds. *PLoSBiol,* 2004, vol. 2 (2), E7
- **VAN DE VIJVER, M.J. et al.** A gene-expression signature as a predictor of survival in breast cancer. *N Engl J Med,* 2002, vol. 347 (25), 1999-2009
- **VAN 'T VEER, L.J. et al.** Gene expression profiling predicts clinical outcome of breast cancer. *Nature,* 2002, vol. 415 (6871), 530-536
- **WANG, Y. et al.** Gene-expression profiles to predict distant metastasis of lymph-node-negative primary breast cancer. *Lancet,* 2005, vol. 365 (9460), 671-679
- **BUYSE, M. et al.** Validation and clinical utility of a 70-gene prognostic signature for women with node-negative breast cancer. *J Natl Cancer Inst,* 2006, vol. 98 (17), 1183-92
- **PAIK, S.** Development and clinical utility of a 21-gene recurrence score prognostic assay in patients with early-stage breast cancer treated with tamoxifen. *Oncologist,* 2007, vol. 12 (6), 631-635
- **PAIK, S. et al.** A multigene assay to predict recurrence of tamoxifen-treated, node-negative breast cancer. *N Engl J Med,* 2004, vol. 351 (27), 2817-2826
- **SOTIRIOU, C. et al.** Gene expression profiling in breast cancer: understanding the molecular basis of histologic grade to improve prognosis. *J Natl Cancer Inst,* 2006, vol. 98 (4), 262-72
- **PAWITAN, Y. et al.** Gene expression profiling spares early-stage breast cancer patients from adjuvant therapy: derived and validated in two population-based cohorts. *Breast Cancer Res,* 2005, vol. 7 (6), R953-964
- **MILLER, L.D. et al.** An expression signature for p53 status in human breast cancer predicts mutation status, transcriptional effects, and patient survival. *Proc Natl AcadSci USA,* 2005, vol. 102 (38), 13550-13555

- **BILD, A.H. et al.** Oncogenic pathway signatures in human cancers as a guide to targeted therapies. *Nature,* 2006, vol. 439 (7074), 353-357
- **TESCHENDORFF, A.E. et al.** A consensus prognostic gene expression classifier for ER positive breast cancer. *Genome Biol,* 2006, vol. 7 (10), R101
- **DESMEDT, C. et al.** Strong time dependence of the 76-gene prognostic signature for node-negative breast cancer patients in the TRANSBIG multicenter independent validation series. *Clin Cancer Res,* 2007, vol. 13 (11), 3207-3214
- **SINGLETON et al.** DICTIONARY OF MICROBIOLOGY AND MOLECULAR BIOLOTY. 1994
- THE CAMBRIDGE DICTIONARY OF SCIENCE AND TECHNOLOGY. 1988
- **HALE ; MARHAM.** THE HARPER COLLINS DICTIONARY OF BIOLOGY
- **MANIATIS et al.** Molecular Cloning: A Laboratory Manual. Cold Spring Harbor Laboratory, 1982
- **SAMBROOK et al.** Molecular Cloning: A Laboratory Manual. Cold Spring Harbor Laboratory Press, 1989
- **DEUTSCHER, M.** Guide to Protein Purification Methods Enzymology. Academic Press. Inc, 1990, vol. 182
- **AUSUBEL et al.** Current Protocols of Molecular Biology. John Wiley and Sons, 1997
- **RUPP ; LOCKER.** *Lab Invest.,* 1987, vol. 56, A67
- **DE ANDRES et al.** *BioTechniques,* 1995, vol. 18, 42044
- **HELD et al.** *Genome Research,* 1996, vol. 6, 986-994
- **T.E. GODFREY et al.** *J. Molec. Diagnostics,* 2000, vol. 2, 84-91
- **K. SPECHT et al.** *Am. J. Pathol.,* 2001, vol. 158, 419-29